# FASCICULE DE BREVET EUROPEEN

(11) **EP 0 800 864 B1**
(45) Date de publication et mention de la délivrance du brevet: **26.09.2001**
(21) Numéro de dépôt: 97400730.4
(22) Date de dépôt: 28.03.1997
(51) Int. Cl.: B01J 37/03, B01J 23/89, B01J 23/75, C07C 1/04

(54) **Procédé de conversion du gaz de synthèse en présence d'un catalyseur a base de cobalt et de tinane**
Verfahren für die Umwandlung von Synthesegas in Gegenwart eines Kobalt und Titan enthaltenden Katalysators
Synthesis gas conversion process using a cobalt and titanium containing catalyst

(30) Priorité: 09.04.1996 FR 9604417
(43) Date de publication de la demande: 15.10.1997
(73) Titulaire: INSTITUT FRANCAIS DU PETROLE, 92502 Rueil-Malmaison (FR); AGIP PETROLI S.p.A., 00142 Roma (IT)
(72) Inventeur: Chaumette, Patrick, 78380 Bougival (FR); Clause, Olivier, 78400 Chatou (FR); Azib, Hedi, 94240 L'Hay Les Roses (FR)
(74) Mandataire: Andreeff, François

(56) Documents cités:
- EP-A- 0 398 420
- EP-A- 0 455 308
- EP-A- 0 629 442
- US-A- 4 176 089
- US-A- 5 169 821

## Description

La présente invention concerne une formulation catalytique, sa préparation et son utilisation dans un procédé de synthèse d'hydrocarbures à partir d'un mélange CO-(CO₂)-H₂, c'est-à-dire d'un mélange CO-H₂ comprenant éventuellement du CO₂ appelé gaz de synthèse, plus particulièrement l'utilisation permettant de réaliser la conversion du gaz de synthèse en un mélange d'hydrocarbures linéaires et saturés essentiellement constitué d'hydrocarbures C₅⁺ (c'est-à-dire possédant au moins 5 atomes de carbone par molécule), ou plus précisement en un mélange d'hydrocarbures essentiellement linéaires et saturés, contenant au moins 25 % poids d'hydrocarbures C₅⁺ par rapport à l'ensemble des hydrocarbures formés.

Il est connu de l'homme du métier que le gaz de synthèse peut être converti en hydrocarbures en présence de catalyseurs contenant des métaux de transition. Cette conversion, opérée à haute température et sous pression, est connue dans la littérature sous le nom de synthèse FISCHER- TROPSCH. Ainsi des métaux du groupe VIII de la classification périodique des éléments tel que le fer, le ruthénium, le cobalt et le nickel catalysent la transformation de mélanges CO-(CO₂)-H₂ en hydrocarbures liquides et/ou gazeux.

Les produits préparés par synthèse FISCHER- TROPSCH en présence de catalyseurs comprenant des métaux du groupe VIII présentent une distribution très large en terme de poids moléculaire. Ainsi seulement une faible proportion des produits obtenus se situent dans la gamme des distillats moyens constitués par des fractions kérosène et gasoil, la (ou les) fraction(s) kérosène étant constituée(s) par un mélange d'hydrocarbures dont les points d'ébullition sont compris entre 140° et 300°C, et la (ou les) fraction(s) gasoil étant constituée(s) par un mélange d'hydrocarbures de points d'ébullition compris entre 180° et 370°C lors d'une distillation atmosphérique telle que réalisée par l'homme du métier sur un brut pétrolier.

Des efforts importants ont été entrepris depuis 1973 afin d'améliorer le rendement en distillats moyens des procédés basés sur la conversion du gaz de synthèse. Ainsi, le catalyseur décrit dans le brevet US-A-5.302.622, comprenant du cobalt, du cuivre et du ruthénium et préparé par gélification, conduit à l'obtention d'un mélange d'hydrocarbures essentiellement linéaires et saturés contenant au moins 80% poids d'hydrocarbures C₅⁺ par rapport à l'ensemble des hydrocarbures formés.

Ces formulations permettent la synthèse d'hydrocarbures essentiellement paraffiniques et linéaires. Toutefois, une proportion importante de ces hydrocarbures est constituée de paraffines de hauts points d'ébullition, c'est-à-dire ayant des points d'ébullition situés au-delà du domaine des distillats moyens.

Il est alors avantageux de traiter ces hydrocarbures à hauts points d'ébullition dans un procédé d'hydrocraquage habituellement utilisé dans le domaine du traitement des coupes lourdes issues d'un brut pétrolier, afin d'améliorer le rendement en distillats moyens.

Il est toutefois dans certains cas avantageux de synthétiser un mélange d'hydrocarbures essentiellement linéaires et saturés contenant généralement moins de 80 % poids d'hydrocarbures C₅⁺, et préférentiellement 25 à 70 % poids d'hydrocarbures C₅⁺. Un tel mélange contient en effet une proportion importante d'hydrocarbures liquides à la température ambiante et à pression atmosphérique.

Après un simple fractionnement (par exemple une distillation atmosphérique), un tel produit essentiellement liquide constitue un brut synthétique qui peut être aisément transporté et/ou mélangé à un brut pétrolier puis traité séparément ou en mélange dans des unités de raffinage conventionnelles.

La demande de brevet EP-A-0 455 308 décrit un procédé de conversion du méthanol en hydrocarbures liquides dans lequel le méthanol est mis en contact avec un catalyseur comprenant un support poreux, du cobalt déposé sur ce support et un promoteur choisi dans le groupe comprenant le zirconium, le titane, le chrome, le ruthénium, le fer, le magnésium, le zinc, le thorium et l'uranium. Lors de la préparation de ce catalyseur, le promoteur peut être déposé en premier sur le support ou au contraire après dépôt du cobalt. Ledit catalyseur est généralement calciné. Il est indiqué que le catalyseur contenant le support, le cobalt et un promoteur, peut être activé sous hydrogène ou un mélange contenant de l'hydrogène à l'issue de sa préparation.
Par contre, il n'est ni indiqué ni suggéré de déposer le promoteur après avoir au moins partiellement réduit le cobalt préalablement déposé.

Le brevet US 4,522,939 décrit un procédé de préparation d'un catalyseur Fischer-Tropsch à base de cobalt et contenant un second métal choisi parmi le zirconium, le titane ou le chrome et supporté sur silice, alumine ou silice-alumine. Ce catalyseur est préparé par imprégnation ou malaxage et le second métal est introduit avant, après ou simultanément à l'introduction du cobalt. D'une manière préférée le catalyseur est calciné après chacune de ces introductions. Il n'est pas prévu d'effectuer une réduction après les diverses étapes d'imprégnation ou de malaxage conduisant à l'obtention de ce catalyseur, une seule réduction étant effectuée après préparation du catalyseur et avant le test catalytique. Ces catalyseurs présentent une désactivation plus ou moins élevée au cours du temps.

Les brevets US-A-4,794,099 et US-A-4,960,801 décrivent des catalyseurs à base de cobalt et éventuellement de rhénium déposé sur des supports à base d'oxyde de titane. Dans le brevet US-A-4,794,099, un précurseur à base de silicium est ajouté à de l'oxyde de titane avant ou après dépôt du cobalt et du rhénium, afin d'améliorer l'activité du catalyseur. Dans le brevet US-A-4,960,801, un liant à base d'aluminium ou de zirconium ou de silicium est ajouté au support à base d'oxyde de titane afin d'augmenter la porosité du support. De même, une seule réduction est prévue après calcination du catalyseur contenant l'ensemble des éléments et avant test catalytique.

La présente invention présente la préparation d'un catalyseur, en synthèse Fischer-Tropsch, dont les performances sont particulièrement stables, et qui conduit après réduction sous hydrogène à la conversion du gaz de synthèse en un mélange d'hydrocarbures essentiellement linéaires et saturés contenant au moins 25% poids d'hydrocarbures C₅⁺ par rapport à l'ensemble des hydrocarbures formés.

La présente invention concerne un procédé de préparation d'un catalyseur comprenant un support choisi dans le groupe formé par au moins un oxyde d'élément choisi dans le groupe formé par les éléments suivants: Si, Al, Ti, Zr, Sn, Zn, Mg, ou Ln (où Ln est une terre rare, c'est-à-dire un élément de numéro atomique compris entre 57 et 71 inclus), de préférence choisi dans le groupe formé par la silice, l'alumine, la zircone et l'oxyde de titane, du cobalt, du titane, au moins un élément additionnel A choisi dans le groupe formé par le cuivre, le ruthénium, le platine, le palladium, le scandium et l'yttrium, et ledit procédé étant caractérisé en ce que la préparation du catalyseur comprend au moins les étapes successives suivantes :
- (1) la formation d'un précurseur comprenant au moins le cobalt, l'élément A et le support,
- (2) la réduction au moins partielle, de préférence sensiblement totale, dudit précurseur en présence d'au moins un composé réducteur, et
- (3) le dépôt sur le précurseur réduit de titane.

Le catalyseur préparé selon l'invention comprend généralement, en % poids d'élément par rapport au poids de support dans le catalyseur, entre 1 et 60 %, de préférence entre 2 et 50 %, du cobalt, entre 0,01 et 20 %, de préférence entre 0,05 et 10 %, d'élément additionnel A et entre 0,01 et 15 %, de préférence entre 0,05 et 5 %, de titane.

L'invention concerne aussi le catalyseur susceptible d'être obtenu par le procédé de préparation selon l'invention, ainsi que l'utilisation dudit catalyseur dans un procédé de synthèse d'hydrocarbures essentiellement linéaires et saturés, contenant au moins 25 % poids d'hydrocarbures C₅⁺ par rapport à l'ensemble des hydrocarbures formés, à partir d'une charge comprenant du monoxyde de carbone CO, de l'hydrogène et éventuellement du bioxyde de carbone CO₂, ladite charge étant appelée gaz de synthèse. Le catalyseur selon l'invention donne des résultats améliorés , en termes de stabilité, par rapport aux catalyseurs de l'art antérieur.

Tout composé à base de titane peut être utilisé dans la présente invention par exemple le titane peut être incorporé sous la forme par exemple d'un halogénure, d'un oxalate, d'un sulfate, d'un alcoxyde tel que l'orthotitanate d'éthyle ou l'orthotitanate d'isopropyle.

Le cobalt et l'élément additionnel A présents dans le précurseur peuvent être introduits, à l'étape (1), par toute méthode connue de l'homme du métier, telle que par exemple l'échange ionique, l'imprégnation à sec, la coprécipitation, la gélification, le mélange ionique ou le greffage de complexes organométalliques.

Parmi ces méthodes, les méthodes d'imprégnation, de coprécipitation, de gélification ou de malaxage sont préférées pour la préparation dudit précurseur à l'étape (1), car elles permettent un contact intime entre le cobalt et l'éventuel élément additionnel A.

En effet, l'emploi des techniques d'imprégnation, de coprécipitation, de gélification ou de malaxage du cobalt, de l'élément A et d'élément du support permet généralement d'obtenir un précurseur de catalyseur de conversion du gaz de synthèse en hydrocarbures, actif et conduisant à la formation d'au moins 25 % poids d'hydrocarbures C₅⁺ par rapport à l'ensemble des hydrocarbures formés.

Une méthode préférée de préparation du précurseur du catalyseur utilisé selon l'invention consiste par exemple à imprégner le support au moyen d'au moins une solution aqueuse (ou dans au moins un solvant approprié) contenant le cobalt et l'élément additionnel A sous forme par exemple d'un halogénure, d'un nitrate, d'un acétate, d'un oxalate, d'un sulfate, d'un complexe formé avec l'acide oxalique et les oxalates, d'un complexe formé avec l'acide citrique et les citrates, d'un complexe formé avec l'acide tartrique et les tartrates, d'un complexe formé avec un autre polyacide ou acide alcool et ses sels, d'un complexe formé avec les acétyl acétonates, et tout autre dérivé inorganique ou organométallique contenant le cobalt et l'élément additionnel A.

Après chaque imprégnation du cobalt et de l'élément additionnel A et éventuellement d'élément choisi dans le groupe formé par les éléments du support sur le support choisi, le précurseur obtenu est ensuite traité thermiquement, c'est-à-dire séché, par tout moyen connu de l'homme du métier, par exemple sous un courant d'azote ou d'air à une température comprise entre 80°C et 200°C, puis calciné par exemple sous un courant d'air ou d'azote à une température comprise par exemple entre 200°C et 800°C.

Il est également possible de préparer le précurseur du catalyseur selon l'invention au moyen de la méthode décrite de façon détaillée dans le brevet US-A- 3 975 302 et qui consiste en la préparation d'une solution d'imprégnation à partir d'un gel solide amorphe et d'alkanolamine, puis à imprégner un support avec cette solution.

Une autre méthode de préparation du précurseur de catalyseur selon l'invention, préférée dans le cadre de la présente invention, consiste en la préparation d'un gel contenant le cobalt, l'élément A et l'(les) élément(s) du support. Cette préparation par gélification se fait selon toute technique connue de l'homme du métier. Cependant, une méthode de préparation par gélification préférée est décrite ci-après.

Une des méthodes de préparation par gélification préférée consiste en la préparation d'un gel obtenu en mélangeant une solution I contenant un composé organométallique, de préférence un alcoxyde d'élément(s) précurseur(s) du support, dissous dans un solvant organique, de préférence un alcool, et une solution aqueuse Il contenant un sel de cobalt, éventuellement au moins un sel d'élément A et un élément du support et contenant également un acide minéral qui accélère la gélification tel que par exemple l'acide nitrique, l'acide chlorhydrique, l'acide sulfurique ou l'acide phosphorique. Lesdits sels de cobalt, de l' élément éventuel A et d'élément(s) du support, sont par exemple des halogénures, des nitrates, des acétates, des oxalates, des sulfates, des complexes formés avec un polyacide ou un acide alcool et ses sels ou des complexes formés avec les acétylacétonates, ou tout autre dérivé inorganique soluble en solution aqueuse. Le mélange des solutions I et II sous agitation en présence dudit acide conduit à l'obtention d'un gel qui est formé en moins de 10 mn et à une température comprise entre 20 et 80°C. Le gel ainsi formé est séparé des solvants résiduels par tout moyen connu de l'homme du métier, par exemple par centrifugation ou filtration, puis séché, par exemple sous un courant d'azote ou d'air à une température comprise entre 80 et 200°C, et enfin calciné, par exemple sous un courant d'air ou d'azote à une température comprise entre 200 et 800°C.

L'étape (2) de réduction du précurseur formé à l'étape (1) est généralement telle que décrite ci-après.

Le précurseur de catalyseur est tout d'abord préréduit par au moins un composé réducteur, par exemple choisi dans le groupe formé par l'hydrogène, le monoxyde de carbone et l'acide formique, éventuellement mis en contact avec un gaz inerte (azote par exemple) dans un rapport molaire composé réducteur/(composé réducteur + gaz inerte) compris entre 0,001:1 à 1:1.

La réduction est menée en phase gazeuse entre 150 et 600°C, de préférence entre 200 et 500°C, entre 0,1 et 10 MPa et à une vitesse volumétrique horaire comprise entre 100 et 40 000 volumes de mélange par volume de catalyseur et par heure. Cette réduction peut également être menée en phase liquide, le catalyseur étant mis en suspension dans un solvant inerte, par exemple une coupe paraffinique.

L'étape (3) de dépôt sur le précurseur réduit de titane est mise en oeuvre selon toute technique connue de l'homme du métier. Une méthode préférée consiste par exemple à imprégner le précurseur réduit au moyen d'au moins une solution aqueuse (ou dans au moins un solvant approprié) contenant du titane, sous forme par exemple d'un halogènure, d'un oxalate, d'un sulfate, d'un alcoxyde tel que l'orthotitanate d'éthyle ou l'orthotitanate d'isopropyle d'un complexe formé avec l'acide oxalique et les oxalates, d'un complexe formé avec l'acide citrique et les citrates, d'un complexe formé avec l'acide tartrique et les tartrates, d'un complexe formé avec un autre polyacide ou acide alcool et ses sels, d'un complexe formé avec les acétyl acétonates, et tout autre dérivé inorganique ou organométallique contenant du titane.

Après dépôt du titane, le catalyseur obtenu est ensuite séché, par tout moyen connu de l'homme du métier, par exemple sous un courant d'azote ou d'air à une température comprise entre 80 et 200°C. Après ce séchage, le catalyseur peut éventuellement être calciné par exemple sous un courant d'air ou d'azote à une température comprise par exemple entre 200 et 800°C.

Le catalyseur est éventuellement mis en forme par tout procédé connu de l'homme du métier, par exemple par extrusion, coagulation en goutte, dragéification ou pastillage. Après cette étape de mise en forme, ledit catalyseur subit éventuellement un séchage dans les conditions opératoires précitées.

Les catalyseurs préparés selon les modes opératoires décrits ci-dessus sont particulièrement bien adaptés pour être utilisés dans les procédés de fabrication d'un mélange d'hydrocarbures essentiellement linéaires et saturés, contenant au moins 25 % en poids d'hydrocarbures C₅⁺ par rapport à l'ensemble des hydrocarbures formés, à partir d'un gaz de synthèse.

Les conditions de mise en oeuvre desdits catalyseurs pour la fabrication d'hydrocarbures selon l'invention sont habituellement les suivantes:

Le catalyseur, chargé dans un réacteur, est soumis à une préréduction avant utilisation, par au moins un composé réducteur, par exemple choisi dans le groupe formé par l'hydrogène, le monoxyde de carbone et l'acide formique, éventuellement mis en contact avec un gaz inerte (azote par exemple) en rapport molaire composé réducteur/(composé réducteur + gaz inerte) compris entre 0,001:1 et 1:1.

La préréduction est menée entre 150 et 600°C, de préférence entre 200 et 500°C, entre 0,1 et 10 MPa et à une vitesse volumétrique horaire comprise entre 100 et 40.000 volumes de mélange par volume de catalyseur et par heure. Cette préréduction peut éventuellement être menée en phase liquide comprenant au moins un hydrocarbure ayant au moins 5, de préférence au moins 10, atomes de carbone par molécule si, par la suite, la réaction de synthèse d'hydrocarbures se déroule en phase liquide comprenant au moins un hydrocarbure ayant au moins 5, de préférence au moins 10, atomes de carbone par molécule.

La conversion du gaz de synthèse en hydrocarbures est ensuite opérée sous une pression totale habituellement comprise entre 0,1 et 15 MPa, et de préférence entre 1 et 10 MPa, la température étant généralement comprise entre 150 et 350°C, et de préférence entre 170 et 300°C.

La vitesse volumétrique horaire est habituellement comprise entre 100 et 20.000 volumes de gaz de synthèse par volume de catalyseur et par heure et de préférence entre 400 et 5 000 volumes de gaz de synthèse par volume de catalyseur et par heure, et le rapport H₂/CO dans le gaz de synthèse est habituellement compris entre 1:2 et 5:1, de préférence entre 1,2:1 et 2,5:1.

Le catalyseur est généralement utilisé en poudre fine calibrée (10-700 mm environ) ou en particules de diamètre équivalent compris entre 2 et 10 mm environ, respectivement en présence d'une phase liquide (dans les conditions opératoires) et d'une phase gazeuse, ou d'une phase gazeuse. La phase liquide peut être constituée par au moins un hydrocarbure ayant au moins 5, de préférence au moins 10, atomes de carbone par molécule.

Les catalyseurs selon l'invention sont particulièrement actifs et stables dans la réaction de synthèse d'hydrocarbures à partir de gaz de synthèse. Finalement, lesdits catalyseurs permettent d'obtenir des hydrocarbures essentiellement linéaires et saturés contenant au moins 25 % d'hydrocarbures C₅⁺ par rapport à l'ensemble des hydrocarbures formés.

Les exemples qui suivent illustrent l'invention.

### Exemple 1 (selon l'invention): catalyseur I

Une solution 1 contenant 130 g d'orthosilicate d'éthyle (T.E.O.S.) dissous dans 50 ml d'éthanol et une solution 2 contenant 46,3 g de nitrate de cobalt hexahydraté, 0,21 g de trichlorure de ruthénium héxamine et 32 g d'acide nitrique concentré dissous dans 80 cm3 d'eau, sont mélangées sous forte agitation à température ambiante.

L'hydrolyse du T.E.O.S. conduit au bout de 15 mn à la formation d'un gel massique contenant les sels de cobalt et de ruthénium.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 1,13g d'orthotitanate d'isopropyle dans 50 ml d'isopropanol.

Après refroidissement à la température ambiante sous hydrogène, le précurseur de catalyseur est ajouté sous inerte à la solution 3, et le mélange est maintenue sous agitation jusqu'à décoloration complète de la solution 3.

Aprés évacuation de l'hydrogène sous inerte et passivation par un mélange à 1% d'oxygène dans de l'azote, une remise à l'air progressive de la solution 3 est effectuée. Le solide est ensuite filtré, séché à l'étuve à 120°C, puis le catalyseur I ainsi obtenu est chargé dans une unité .

### Exemple 2 (selon l'invention): catalyseur J

Une solution 1 contenant 130 g d'orthosilicate d'éthyle (T.E.O.S.) dissous dans 50 ml d'éthanol et une solution 2 contenant 46,3 g de nitrate de cobalt hexahydraté, 0,14 g de nitrate de cuivre trihydraté et 32 g d'acide nitrique concentré dissous dans 80 cm3 d'eau, sont mélangées sous forte agitation à température ambiante.

L'hydrolyse du T.E.O.S. conduit au bout de 19 mn à la formation d'un gel massique contenant les sels de cobalt et de cuivre.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 1,13g d'orthotitanate d'isopropyle dans 50 ml d'isopropanol.

Après refroidissement à la température ambiante sous hydrogène, le précurseur du catalyseur est ajouté sous inerte à la solution 3, et le mélange est maintenue sous agitation jusqu'à décoloration complète de la solution 3.

Après évacuation de l'hydrogène sous inerte et passivation par un mélange à 1% d'oxygène dans de l'azote, une remise à l'air progressive de la solution 3 est effectuée. Le solide obtenu est ensuite filtré, séché à l'étuve à 120°C, puis le catalyseur J ainsi obtenu est chargé dans une unité .

### Exemple 3 (selon l'invention): catalyseur K

Une solution 1 contenant 130 g d'orthosilicate d'éthyle (T.E.O.S.) dissous dans 50 ml d'éthanol et une solution 2 contenant 27,8 g de nitrate de cobalt hexahydraté, 0,04 g de trichlorure de ruthénium héxamine, et 32 g d'acide nitrique concentré dissous dans 80 cm3 d'eau, sont mélangées sous forte agitation à température ambiante.

L'hydrolyse du T.E.O.S. conduit au bout de 19 mn à la formation d'un gel massique contenant les sels de cobalt et de ruthénium.

Le gel obtenu est séparé des eaux mères, séché à l'étuve entre 40°C et 120°C, puis calciné à 600°C sous air.

Le précurseur de catalyseur ainsi obtenu est réduit, à pression atmosphérique, par un mélange d'hydrogène et d'azote contenant 6% d'hydrogène dans l'azote entre la température ambiante et 240°C, puis sous hydrogène pur entre 240°C et 450°C.

Une solution 3 est préparée, qui contient 0,11 g d'orthotitanate d'isopropyle dans 50 ml d'isopropanol sous atmosphère inerte.

Après refroidissement à la température ambiante sous hydrogène, le précurseur est ajouté sous inerte à la solution 3, et le mélange est maintenue sous agitation jusqu'à décoloration complète de la solution 3.

Après évacuation de l'hydrogène sous inerte et passivation par un mélange à 1% d'oxygène dans de l'azote, une remise à l'air progressive de la solution 3 est effectuée. Le solide obtenu est ensuite filtré, séché à l'étuve à 120°C, puis le catalyseur K ainsi obtenu est chargé dans une unité .

### Exemple 4 (comparatif): catalyseur L

Une solution 1 contenant 130 g d'orthosilicate d'éthyle (T.E.O.S.) dissous dans 50 ml d'éthanol et une solution 2 contenant 46,3 g de nitrate de cobalt hexahydraté, 0,21 g de trichlorure de ruthénium héxamine et 32 g d'acide nitrique concentré dissous dans 80 cm³ d'eau, sont mélangées sous forte agitation à température ambiante.

L'hydrolyse du T.E.O.S. conduit au bout de 17 mn à la formation d'un gel massique contenant les sels de cobalt et de ruthénium.

Le gel obtenu est séparé des eaux mères, séché à l'étuve entre 40°C et 120°C, puis calciné à 600°C sous air. Le catalyseur L ainsi obtenu est chargé dans une unité .

### Exemple 5 (comparatif): catalyseur M

Une solution 1 contenant 130g d'orthosilicate d'éthyle (T.E.O.S.) et 1,13 g d'orthotitanate d'isopropyle dissous dans 50 ml d'éthanol et une solution 2 contenant 46,3 g de nitrate de cobalt hexahydraté, 0,21 g de trichlorure de ruthénium hexamine et 32 g d'acide nitrique dissous dans 80 cm³ d'eau, sont mélangés sous forte agitation à température ambiante.

L'hydrolyse conduit au bout de 15 minutes à un gel de silice contenant les sels de cobalt, ruthénium et un composé hydrolysé du titane.

Le catalyseur M ainsi obtenu est chargé dans une unité.

### Exemple 6 (comparatif): catalyseur N

Le même gel qu'à l'exemple 1 est préparé, séché puis calciné, mais il n'est pas réduit.

Puis une solution 3 est préparée qui contient 1,13 g d'orthotitanate d'isopropyle dans 20 ml d'isopropanol. Le solide est ensuite évaporé sous vide jusqu'à 80° C, puis séché à l'étuve entre 40° C et 120° C puis calciné sous air. Le catalyseur N obtenu est chargé dans une unité.

**TABLEAU 1 :**

| **COMPOSITION DES CATALYSEURS *** | | | | |
|---|---|---|---|---|
| **EXEMPLE** | **CATALYSEUR** | **Co** | **A** | **Ti** |
| **1** | **I (inv.)** | **25** | **Ru : 0,19** | **0,5** |
| **2** | **J (inv.)** | **25** | **Cu : 0,10** | **0,5** |
| **3** | **K (inv.)** | **15** | **Ru : 0,11** | **0,3** |
| **4** | **L (comp.)** | **25** | **Ru : 0,19** | **0** |
| **5** | **M (comp.)** | **25** | **Ru : 0,19** | **0,5** |
| **6** | **N (comp.)** | **25** | **Ru : 0,19** | **0,5** |

| | | | | |
|---|---|---|---|---|
| * exprimée en %poids de chaque élément par rapport au poids de silice | | | | |

### Exemple 7: Tests catalytiques

Les catalyseurs I, J, K, L, M, N dont les préparations sont décrites dans les exemples 1 à 6 ci-dessus ; sont testés en lit fixe phase gazeuse dans une unité fonctionnant en continu et opérant sur 20 cm3 de catalyseur.

Les catalyseurs I à N sont préalablement réduits in-situ jusqu'à 240°C par un mélange d'hydrogène et d'azote contenant 6% d'hydrogène dans l'azote, puis par de l'hydrogène pur jusqu'à 450°C, à la pression atmosphérique. Les conditions de test des catalyseurs sont les suivantes:
- Température 240°C
- Pression 2MPa
- Vitesse volumique horaire (V.V.H.) : 2000 h-1
- Rapport molaire H2:CO= 2:1

**TABLEAU 2 :**

| **CONVERSION DU GAZ DE SYNTHESE EN HYDROCARBURES** | | | | | |
|---|---|---|---|---|---|
| CATALYSEUR | CONV. CO (%vol.) | DISTRIBUTION DES HYDROCARBURES FORMES (%Pds) | | | VARIATION DE CONVERSION |
| | après 100 h | C₁ | C₂-C₄ | C₅⁺ | *D Conv CO/1000 h(%) |
| I | 60 | 5,7 | 2,5 | 91,8 | + 9 |
| J | 65 | 6,3 | 3,4 | 90,3 | - 8 |
| K | 55 | 23,2 | 26,2 | 50 | - 5 |
| L | 45 | 7,5 | 3,6 | 89,9 | - 30 |
| M | 50 | 8,5 | 4,2 | 87,5 | - 25 |
| N | 45 | 7,2 | 3,1 | 89,7 | - 17 |

| | | | | | |
|---|---|---|---|---|---|
| * = Conv CO/1000 h(%) - conv. CO (initiale) (%) | | | | | |

## Revendications

1. Procédé de préparation d'un catalyseur comprenant un support choisi dans le groupe formé par au moins un oxyde d'élément choisi dans le groupe formé par les éléments suivants: Si, Al, Ti, Zr, Sn, Zn, Mg, Ln (où Ln est une terre rare) du cobalt, du titane, au moins un élément additionnel A choisi dans le groupe formé par le cuivre, le ruthénium, le platine, le palladium, le scandium et l'yttrium, et **caractérisé en ce qu'**il comprend au moins les étapes successives suivantes :
- (1) la formation d'un précurseur comprenant le cobalt, l'élément A et le support
- (2) la réduction au moins partielle dudit précurseur en présence d'au moins un composé réducteur, et
- (3) le dépôt sur le précurseur réduit de titane.

2. Procédé de préparation selon la revendication 1 tel que le catalyseur comprend, en % poids d'élément par rapport au poids de support, entre 1 et 60 % de cobalt, entre 0,01 et 20 % d'élément additionnel A et entre 0,01 et 15 % de titane.

3. Procédé de préparation selon l'une des revendications 1 ou 2 tel que la réduction de l'étape (2) est sensiblement totale.

4. Catalyseur susceptible d'être obtenu par le procédé de préparation selon l'une des revendications 1 à 3.

5. Procédé de synthèse d'hydrocarbures essentiellement linéaires et saturés, contenant au moins 25 % poids d'hydrocarbures C₅⁺ par rapport à l'ensemble des hydrocarbures formés, à partir d'un gaz de synthèse CO-(CO₂)-H₂, en présence d'un catalyseur selon la revendication 4, tel que la conversion du gaz de synthèse en hydrocarbures est opérée sous une pression totale comprise entre 0,1 et 15 MPa, la température étant comprise entre 150 et 350 °C, la vitesse volumique horaire étant comprise entre 100 et 20 000 volumes de gaz de synthèse par volume de catalyseur et par heure, et le rapport molaire H₂/CO dans le gaz de synthèse étant compris entre 1:2 et 5:1.

6. Procédé selon la revendication 5 dans lequel le catalyseur est soumis à une préréduction avant utilisation, ladite préréduction du catalyseur étant effectuée par au moins un composé réducteur, éventuellement mis en contact avec un gaz inerte en rapport molaire composé réducteur/(composé réducteur + gaz inerte) compris entre 0,001:1 et 1:1, la préréduction étant menée entre 150°C et 600°C, de préférence entre 200 et 500°C, entre 0,1 et 10 MPa et à une vitesse volumétrique horaire de 100 à 40 000 volumes de mélange par volume de catalyseur et par heure.

## Claims

1. A process for the preparation of a catalyst comprising a support selected from the group consisting of at least one oxide of an element selected from the group consisting of the following elements: Si, Al, Ti, Zr, Sn, Zn, Mg or Ln (where Ln is a rare earth), cobalt, and titanium, at least one additional element A selected from the group consisting of copper, ruthenium, platinum, palladium, scandium and yttrium, and **characterized in that** it comprises at least the following successive steps:
(1) formation of a precursor comprising cobalt, element A and the support;
(2) at least partial reduction of said precursor in the presence of at least one reducing compound; and
(3) depositing titanium on the reduced precursor.

2. A preparation process according to claim 1, in which the catalyst comprises, as a % by weight of the element with respect to the weight of the support, between 1% and 60% of cobalt, between 0.01% and 20% of additional element A and between 0.01% and 15% of titanium.

3. A preparation process according to claim 1 or claim 2, in which the reduction of step (2) is substantially complete.

4. A catalyst obtained from the preparation process of any one of claims 1 to 3.

5. A process for the synthesis of essentially linear and saturated hydrocarbons containing at least 25% by weight of C₅⁺ hydrocarbons with respect to the total amount of hydrocarbons formed, from a synthesis gas CO-(CO₂)-H₂, in the presence of a catalyst according to claim 4, in which the synthesis gas is converted to hydrocarbons at a total pressure which is in the range 0.1 to 15 MPa, a temperature which is in the range 150°C to 350°C, an hourly space velocity which is in the range 100 to 20000 volumes of synthesis gas per volume of catalyst per hour, and an H₂/CO molar ratio in the synthesis gas in the range 1:2 to 5:1.

6. A process according to claim 5, in which the catalyst is pre-reduced before use, said catalyst pre-reduction being carried out by at least one reducing compound, optionally brought into contact with an inert gas in a reducing compound/(reducing compound + inert gas) molar ratio which is in the range 0.001:1 to 1:1, pre-reduction being carried out between 150°C and 600°C, preferably between 200°C and 500°C, between 0.1 MPa and 10 MPa and at an hourly space velocity of 100 to 40000 volumes of mixture per volume of catalyst per hour.

## Patentansprüche

1. Herstellungsverfahren eines Katalysators, der einen Träger umfasst, der in der durch wenigstens ein Oxid eines Elements gebildeten Gruppe gewählt ist, das in der durch die folgenden Elemente gebildeten Gruppe gewählt ist: Si, Al, Ti, Zr, Sn, Zn, Mg, Ln (wobei Ln eine Seltenerde ist), Cobalt, Titan, wenigstens ein zusätzliches Element A, das aus der durch Kupfer, Rhenium, Platin, Palladium, Scandium und Yttrium gebildeten Gruppe gewählt ist, und
**dadurch gekennzeichnet, dass** es wenigstens die folgenden Stufen umfasst:
- (1) Bildung eines Vorläufers, der Cobalt, das Element A und den Träger umfasst
- (2) wenigstens partielle Reduktion dieses Vorläufers in Gegenwart wenigstens einer reduzierenden Verbindung und
- (3) Abscheiden von Titan auf dem reduzierten Vorläufer

2. Herstellungsverfahren nach Anspruch 1 derart, dass der Katalysator in Gew.-% des Elements im Gewichtsverhältnis zum Träger zwischen 1 und 60% Cobalt, zwischen 0,01 und 20% des zusätzlichen Elements A und zwischen 0,001 und 15% Titan umfasst.

3. Herstellungsverfahren nach einem der Ansprüche 1 oder 2, derart, dass die Reduktion der Stufe (2) im wesentlichen vollständig ist.

4. Katalysator, erhältlich durch das Herstellungsverfahren nach einem der Ansprüche 1 bis 3.

5. Syntheseverfahren von im wesentlichen linearen und gesättigten Kohlenwasserstoffen, die wenigstens 25 Gew.-% C₅⁺ Kohlenwasserstoffe im Verhältnis zur Gesamtheit der gebildeten Kohlenwasserstoffe enthalten, ausgehend von Synthesegas CO-(CO₂)-H₂ in Gegenwart eines Katalysators nach Anspruch 4, derart, dass die Umwandlung des Synthesegases zu Kohlenwasserstoffen unter einem Gesamtdruck zwischen 0,1 und 15 MPa betrieben wird, wobei die Temperatur zwischen 150 und 350°C liegt, die stündliche Raumgeschwindigkeit zwischen 100 und 20000 Volumen Synthesegas pro Katalysatorvolumen und pro Stunde und das molare Verhältnis H₂/CO in dem Synthesegas zwischen 1:2 und 5:1 liegt.

6. Verfahren nach Anspruch 5, wobei der Katalysator einer Vorreduktion vor dem Gebrauch unterzogen wird, wobei diese Vorreduktion des Katalysators durch wenigstens eine reduzierende Verbindung, gegebenenfalls Kontaktieren mit einem Inertgas, im molaren Verhältnis reduzierende Verbindung/(reduzierende Verbindung + Inertgas), das zwischen 0,001:1 und 1:1 liegt, durchgeführt wird, wobei die Vorreduktion zwischen 150°C und 600°C, vorzugsweise zwischen 200 und 500°C, zwischen 0,1 und 10 MPa und bei einer stündlichen Volumengeschwindigkeit von 100 bis 40000 Mischungsvolumen pro Katalysatorvolumen und pro Stunde geführt wird.
